# EUROPEAN PATENT APPLICATION

(11) **EP 3 050 498 A1**
(43) Date of publication of application: **03.08.2016**
(21) Application number: 14849678.9
(22) Date of filing: 22.09.2014
(51) Int. Cl.: A61B 5/00, A61B 5/01, G01D 9/00, G06Q 50/24

(54) **BIOLOGICAL INFORMATION MANAGEMENT SYSTEM AND BIOLOGICAL INFORMATION MANAGEMENT METHOD**

(30) Priority: 25.09.2013 JP 2013198321
(71) Applicant: Terumo Kabushiki Kaisha, Tokyo 151-0072 (JP)
(72) Inventor: TOUNOOKA, Yuuya, Ashigarakami-gun Kanagawa 259-0151 (JP)
(74) Representative: Casalonga
(86) International application number: PCT/JP2014/075039
(87) International publication number: WO 2015/046130

(57) **Abstract**

To provide a biological information management system which allows a biological information acquisition device for acquiring biological information and the like to provide acquired biological information to a plurality of biological information management devices, such as servers, and which prevents the biological information in the biological information acquisition device to be mistaken for information of another patient.

A biological information management system (1) includes a biological information acquisition device (10) for storing biological information of a subject person, and a biological information management device (50) for managing the biological information together with subject person information, where the biological information acquisition device includes a biological information storage unit for storing the biological information, causes the biological information storage unit (16) to store new biological information, and deletes already registered biological information from the biological information storage unit, and where the biological information acquisition device provides the biological information in response to a request from the biological information management device, and acquires the subject person information of the provided biological information from the biological information management device.

## Description

### Technical Field

The present invention relates to a biological information management system and a biological information management method for acquiring and managing biological information, such as body temperature, of a patient, for example.

### Background Art

Conventionally, a weight scale is used to measure biological information, such as weight, of a patient at a hospital or the like, and the weight scale or the like is provided with a display, and the measured weight is displayed on the display.

However, since a large number of patients come to the hospital or the like, the weight or the like displayed on the display of the weight scale is possibly seen by other people. Accordingly, it is proposed to erase the weight display on such a display (for example, Patent Literature 1).

Furthermore, weight scales, thermometers and the like used at a hospital or the like assume use by many patients, and thus, if body temperature or the like of each patient is stored for a long time in the thermometer itself or the like, it may be handled as the body temperature of another patient by mistake.

Accordingly, for example, a method is adopted according to which an electronic medical record server or the like for managing body temperature information or the like is installed at a hospital or the like, and when body temperature information is transmitted from a thermometer to the electronic medical record server or the like, the body temperature information in the thermometer is erased, and the body temperature information is prevented from remaining in the thermometer.

### Citation List

### Patent Literature

Patent Literature 1: JP 2013-81754 A

### Summary of Invention

### Technical Problem

However, in many cases, a plurality of biological information management devices, such as electronic medical record servers, are present at one same hospital or the like, and in such a case, if biological information, such as body temperature information, is stored in one electronic medical record server, and then a process of deleting the body temperature information from a biological information acquisition device, such as a thermometer, is performed, there is a problem that other biological information management devices cannot acquire the biological information, such as the body temperature information, in the biological information acquisition device.

On the other hand, if body temperature information or the like measured in the past is indefinitely stored in a biological information acquisition, such as a thermometer, there is a problem that the information is mistaken for the biological information, such as body temperature information, of another patient.

Accordingly, the present invention has its object to provide a biological information management system and a biological information management method which allow a biological information acquisition device, such as a thermometer, for acquiring biological information, such as body temperature information, to provide acquired biological information to a plurality of biological information management devices, such as servers, and which may prevent biological information stored in the biological information acquisition device to be mistaken for the information of another patient.

### Solution to Problem

In the present invention, the above described object is achieved by a biological information management system including: a biological information acquisition device for storing biological information acquired from a subject person; and a biological information management device for managing the biological information together with subject person information, wherein the biological information acquisition device includes a biological information storage unit for storing the biological information, and when new biological information is acquired, the biological information acquisition device causes the biological information storage unit to store the new biological information, and deletes the biological information that is already registered from the biological information storage unit, and wherein the biological information acquisition device is configured to provide the biological information in response to a request from the biological information management device, and is configured to acquire the subject person information of the provided biological information from the biological information management device.

According to the configuration described above, the biological information acquisition device, such as a thermometer, includes the biological information storage unit for storing the biological information, such as body temperature information, and when new body temperature information or the like is acquired, the biological information acquisition device causes the biological information storage unit to store the new body temperature information, and deletes the body temperature information or the like that is already registered from the biological information storage unit.

Accordingly, even after providing the body temperature information or the like to the biological information management device, such as an electronic medical record server, the thermometer or the like holds the provided body temperature information or the like until new body temperature information or the like is acquired, and thus the thermometer may provide the body temperature information or the like when afterwards there is a request for provision of the same from another electronic medical record server or the like.

Furthermore, when the thermometer or the like acquires new body temperature information or the like, old body temperature information which was already registered is deleted, and thus biological information, such as the body temperature information, which is personal information of a subject person is not held for an unnecessarily long time, and also the biological information to be held is limited to the latest biological information, and therefore leakage and the like of personal information, i.e. biological information, may be prevented.

Moreover, according to the configuration described above, the biological information acquisition device, such as the thermometer, is configured to provide the biological information, such as the body temperature information, in response to a request from the biological information management device, such as the electronic medical record server, and is configured to acquire the subjectpersoninformation (patient information and the like), such as the provided body temperature information, from the electronic medical record server or the like.

Accordingly, the thermometer or the like may acquire reliable certain patient information or the like from the electronic medical record server or the like without possessing the subject person information beforehand, such as the patient information, and the manufacturing cost of the thermometer or the like may be reduced.

Furthermore, at this time, the thermometer holds only the latest body temperature information or the like, and thus there is no confusion about which body temperature information or the like is associated with the patient information or the like acquired from the electronic medical record server or the like, and there is no mix-up of the patient information or the like and the body temperature information or the like.

Therefore, when the thermometer or the like is afterwards requested by another electronic medical record server or the like to provide the body temperature information or the like, the patient information or the like may be provided at the same time together with the body temperature information or the like, and also the accuracy of these pieces of information may be guaranteed.

Preferably, the biological information acquisition device is configured to provide the biological information to a plurality of the biological information management devices, and when there is a request for the biological information from another biological information management device after the subject person information is acquired from one biological information management device, the biological information acquisition device provides the biological information together with the acquired subject person information.

According to the configuration described above, when there is a request for the body temperature information or the like from another electronic medical record server or the like after the subject person information, such as the patient information, is acquired from one biological information management device, such as the electronic medical record server, the biological information acquisition device, such as a thermometer, provides the body temperature information or the like together with the acquired patient information or the like.

Accordingly, occurrence of a situation where the patient information or the like and the body temperature information or the like stored in a plurality of different electronic medical record servers or the like are different may be prevented.

In the present invention, the above described object is achieved by a biological information management method that uses a biological information management system including biological information acquisition device for storing biological information acquired from a subject person and a biological information management device for managing the biological information together with subject person information, wherein the biological information acquisition device includes a biological information storage unit for storing the biological information, and when new biological information is acquired, the biological information acquisition device causes the biological information storage unit to store the new biological information, and deletes the biological information that is already registered from the biological information storage unit, and wherein the biological information acquisition device is configured to provide the biological information in response to a request from the biological information management device, and is configured to acquire the subject person information of the provided biological information from the biological information management device.

### Advantageous Effects of Invention

As described above, according to the present invention, there may be provided a biological information management system and a biological information management method which allow a biological information acquisition device, such as a thermometer, for acquiring biological information or the like, such as body temperature information, to provide acquired biological information to a plurality of biological information management devices, such as servers, and which may prevent biological information stored in the biological information acquisition device to be mistaken for the information of another patient or the like.

### Brief Description of Drawings

Fig. 1 is a schematic diagram that illustrates a biological information management system according to an embodiment of the present invention.
Fig. 2 is a schematic block diagram that illustrates main structures of a thermometer in Fig. 1.
Fig. 3 is a schematic block diagram that illustrates main structures of a ward terminal in Fig. 1.
Fig. 4 is a schematic block diagram that illustrates main structures of a ward electronic medical record server in Fig. 1.
Fig. 5 is a schematic flow chart that illustrates main operation and the like of a biological information management system illustrated in Fig. 1.
Fig. 6 is another schematic flow chart that illustrates main operation and the like of the biological information management system illustrated in Fig. 1.

### Description of Embodiments

Hereinafter, a preferred embodiment of the present invention will be described in detail with reference to the appended drawings.

Additionally, the embodiment described below is a preferred concrete example of the present invention, and is limited in various technically desirable ways, but the scope of the present invention is not limited to these modes unless the present invention is specifically limited in the following description.

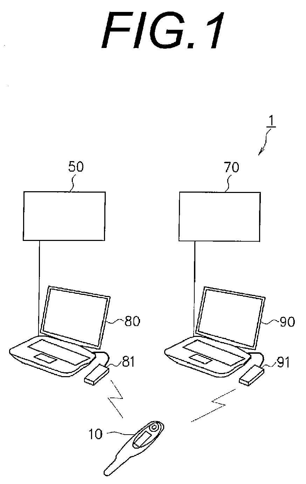

Fig. 1 is a schematic diagram that illustrates a biological information management system 1 according to an embodiment of the present invention.

As illustrated in Fig. 1, the biological information management system 1 includes a thermometer 10, which is an example of a biological information acquisition device.

The thermometer 10 is used, at a hospital or the like, by an unspecified large number of subj ect persons such as patients, for example.

Moreover, after acquiring body temperature information, which is an example of biological information of a patient, the thermometer 10 transmits, by communication, the body temperature information to a plurality of biological information management devices such as a "ward electronic medical record server 50" and an "outpatient electronic medical record server 70".

Specifically, the ward electronic medical record server 50 is connected to a "ward terminal 80", which is a computer installed at each ward, and the ward terminal 80 includes a "ward terminal read/write device 81" which is capable of near field communication that uses electromagnetic induction or the like.

Furthermore, the "outpatient electronic medical record server 70" is connected to an "outpatient terminal 90", which is a computer installed at each outpatient clinic, and the "outpatient terminal 90" includes an "outpatient terminal read/write device 91" which is capable of near field communication that uses electromagnetic induction or the like.

For its part, the thermometer 10 includes, as will be described later, a "thermometer-side communication device 11", and the thermometer-side communication device 11 is also capable of near field communication that uses electromagnetic induction or the like, and by bringing the thermometer 10 close to the "ward terminal read/write device 81" or the "outpatient terminal read/write device 91", the thermometer 10 may perform communication with the "ward terminal read/write device 81" or the "outpatient terminal read/write device 91", and thus communication with the "ward terminal 80" and the "outpatient terminal 90" is also made possible.

Also, as described above, since the "ward terminal 80" and the "outpatient terminal 90" are connected, respectively, to the "ward electronic medical record server 50" and the "outpatient electronic medical record server 70" in a manner capable of communication, body temperature information or the like acquired by the "ward terminal 80" and the "outpatient terminal 90" from the thermometer 10 is transmitted to the "ward electronic medical record server 50" and the "outpatient electronic medical record server 70".

Additionally, the "ward electronic medical record server 50" and the "outpatient electronic medical record server 70" in Fig. 1 are independent from each other and do not communicate with each other to share data, and acquire body temperature information and the like independently of each other.

Furthermore, the "ward electronic medical record server 50" and the "outpatient electronic medical record server 70" store acquired body temperature information or the like (measurement result, measurement date/time, and the like) in association with patient information (for example, patient name, patient ID, date/time of registration as patient, and the like) which is subject person information of a patient whose body temperature has been measured. Additionally, at this time, data of time of registration in the "ward electronic medical record server 50" or the like is also stored in association.

Accordingly, patient information of each patient, body temperature information and the like of each patient, and other pieces of related information are associated with one another in the "ward electronic medical record server 50" and the "outpatient electronic medical record server 70".

The thermometer 10, the ward electronic medical record server 50, the outpatient electronic medical record server 70, the ward terminal 80, and the outpatient terminal 90 illustrated in Fig. 1 include a computer, and the computer includes a central processing unit (CPU), a random access memory (RAM), a read only memory (ROM) and the like, not illustrated, and these are interconnected by a bus.

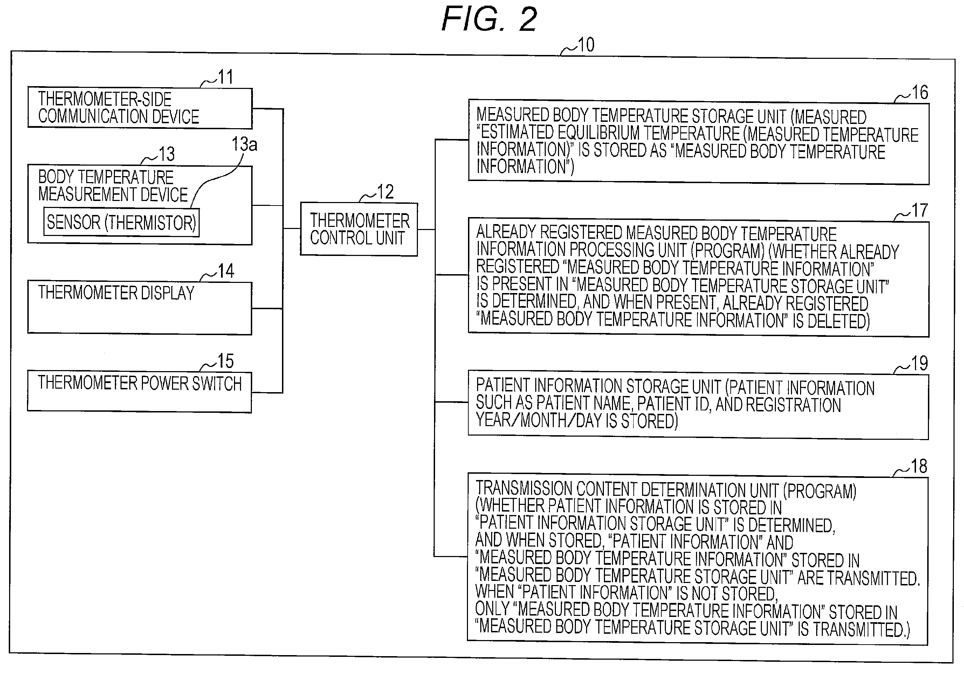

Fig. 2 is a schematic block diagram that illustrates main structures of the thermometer 10 in Fig. 1. As shown in Fig. 2, the thermometer 10 includes a "thermometer control unit 12", and the thermometer control unit 12 controls the "thermometer-side communication device 11", a "body temperature measurement device 13" that includes a "sensor (thermistor) 13a", a "thermometer display 14" for displaying measured body temperature and the like, the "thermometer power switch 15", and the like, and also controls various storage units and processing units (programs) illustrated in Fig. 2 and the like, and details thereof will be given later.

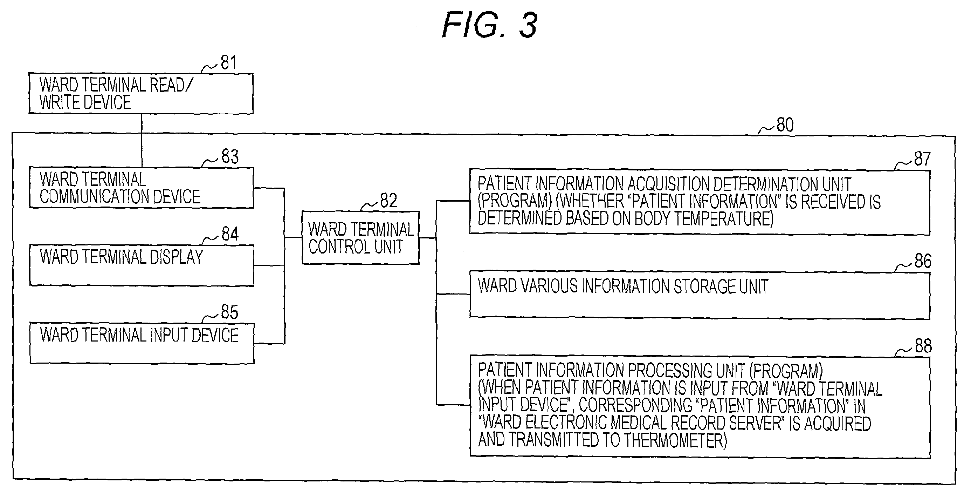

Fig. 3 is a schematic block diagram that illustrates main structure of the ward terminal 80 in Fig. 1. Additionally, the outpatient terminal 90 in Fig. 1 has approximately the same configuration, and description thereof is omitted.

As illustrated in Fig. 3, the ward terminal 80 includes a "ward terminal control unit 82", and the ward terminal control unit 82 controls a "ward terminal communication device 83" that is capable of communicating with the ward terminal read/write device 81 in Fig. 1, a "ward terminal display 84" for displaying various pieces of information, and a "ward terminal input device 85" for inputting various pieces of information, and also controls "ward various information storage unit 86" for storing various pieces of information, a "patient information acquisition determination unit (program) 87", and a "patient information processing unit (program) 88".

Additionally, details of the "patient information acquisition determination unit (program) 87" and the "patient information processing unit (program) 88" will be given later.

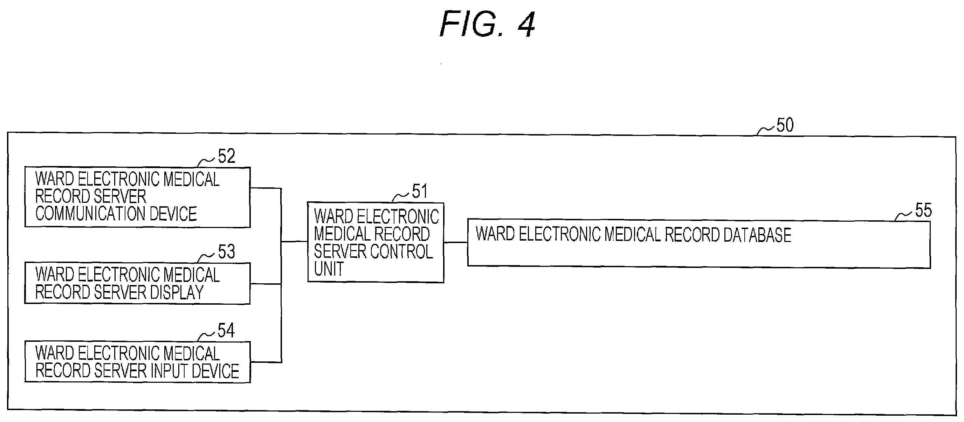

Fig. 4 is a schematic block diagram that illustrates main structures of the ward electronic medical record server 50 in Fig. 1. Additionally, the outpatient electronic medical record server 70 in Fig. 1 has approximately the same configuration, and description thereof is omitted.

As illustrated in Fig. 4, the ward electronic medical record server 50 includes a "ward electronic medical record server control unit 51", and the ward electronic medical record server control unit 51 controls a "ward electronic medical record server communication device 52" for communicating with the ward terminal 80 in Fig. 1, a "ward electronic medical record server display 53" for displaying various pieces of information, and a "ward electronic medical record server input device 54" for inputting various pieces of information, and also controls a "ward electronic medical record database 55" storing, in association with one another, the body temperature information, the patient information and the like described above.

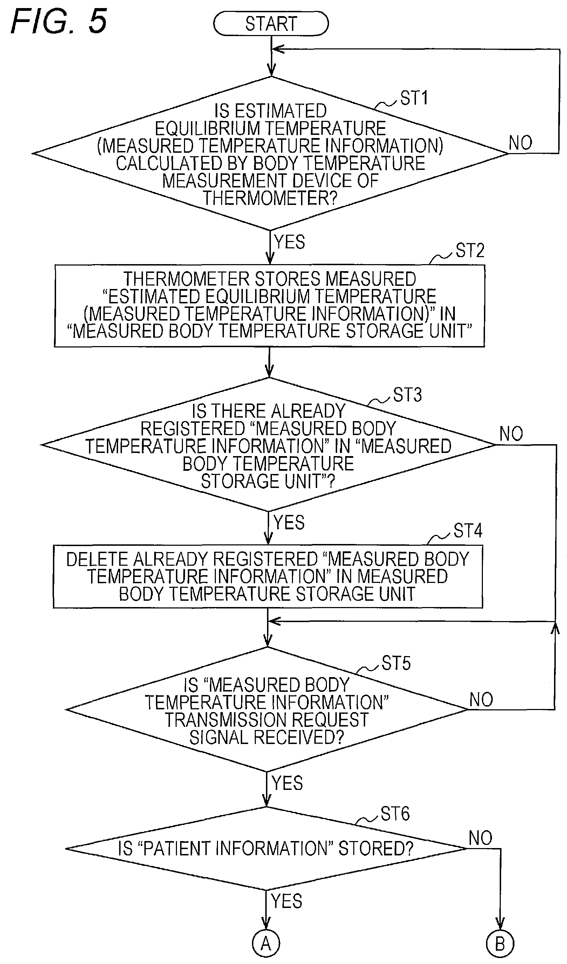

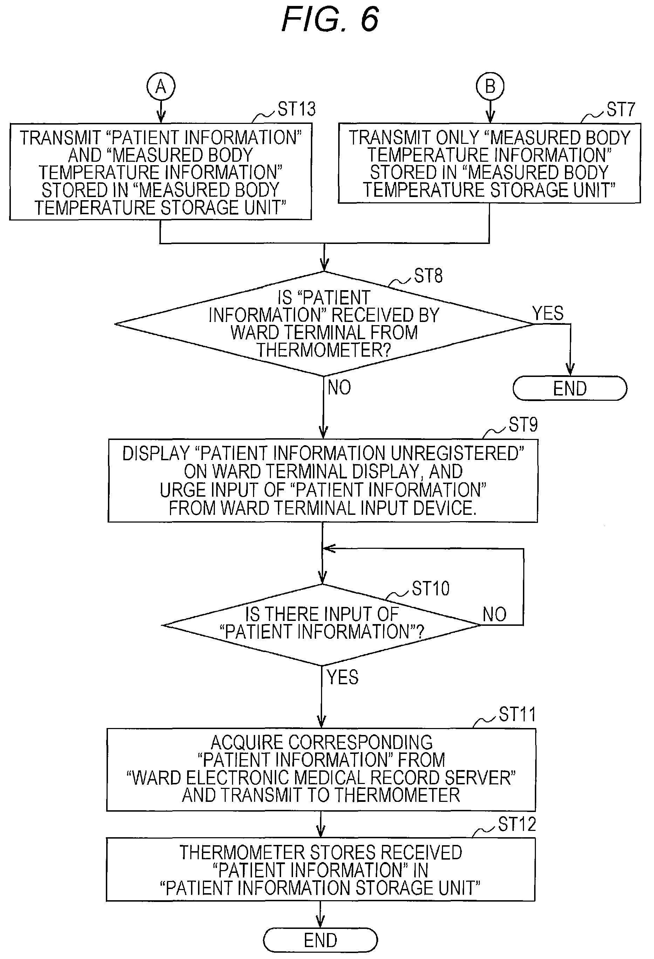

Figs. 5 and 6 are schematic flow charts that illustrate main operation and the like of the biological information management system 1 illustrated in Fig. 1.

In the present embodiment, description is given by using an example where, after a patient has checked in to the hospital and had his/her body temperature measured at the ward by using the thermometer 10, the body temperature information is stored in the ward electronic medical record server 50, and then the same body temperature information is stored in the outpatient electronic medical record server 70.

First, the patient measures his/her body temperature at his/her bed in the ward by using the thermometer 10.

Then, the process proceeds to step ST (hereinafter abbreviated as "ST") 1 in Fig. 5. In ST1, whether an estimated equilibrium temperature (measured temperature information) is calculated by the body temperature measurement device 13 of the thermometer 10 or not is determined.

Then, when it is determined that calculation has been performed, the thermometer 10 stores the measured "estimated equilibrium temperature (measured temperature information)" as "measured body temperature information" in a "measured body temperature storage unit 16", which is an example of the biological information storage unit in Fig. 2.

Then, the process proceeds to ST3. In ST3, an "already registered measured body temperature information processing unit (program) 17" in Fig. 2 is operated, and whether already registered "measured body temperature information" is stored in the "measured body temperature storage unit 16" or not is determined.

If, in ST3, already registered "measured body temperature information" is stored, this "measured body temperature information" is deleted (ST4).

Accordingly, after new measured body temperature information is acquired, old measured body temperature information which was already registered is deleted from the "measured body temperature storage unit 16" of the thermometer 10, and thus measured body temperature information, which is personal information of a patient, is not held by the thermometer 10 for an unnecessarily long time.

Also, the measured body temperature information that is held by the thermometer 10 is limited to the latest measured body temperature information, and thus leakage and the like of personal information, i.e. the measured body temperature information, may be prevented.

The measurement process of body temperature of a patient by the thermometer 10 is then ended. Next, at the bed in the ward, a medical worker such as a nurse brings the "ward terminal 80" illustrated in Fig. 1, together with the "ward terminal read/write device 81", to the bedside of the patient.

The nurse brings the thermometer 10 of the patient whose body temperature has been measured close to the ward terminal read/write device 81.

Then, the process proceeds to ST5. In ST5, the thermometer 10 determines whether a signal of "measured body temperature information transmission request" is received from the ward terminal 80 or not.

If it is determined in ST5 that a signal of "measured body temperature information transmission request" is received from the ward terminal 80, the process proceeds to ST6. In ST6, a "transmission content determination unit (program) 18" in Fig. 2 is executed, and a "patient information storage unit 19", in Fig. 2, of the thermometer 10 is referred to.

The "patient information storage unit 19" stores "patient information (patient name, patient ID, patient registration year/month/day, etc.)", related to the patient, which has been acquired from the "ward electronic medical record server 50" or the like.

In the present step, the "patient information storage unit 19" is referred to, and whether patient information is already stored or not is determined.

If, in ST6, patient information is not stored, the process proceeds to ST7 in Fig. 6. In ST7, the thermometer 10 transmits, to the "ward terminal 80" side, only the "measured body temperature information" that is stored in the "measured body temperature storage unit 16".

Next, the process proceeds to ST8. In ST8, the "patient information acquisition determination unit (program) 87"inFig. 3 is executed, and whether "patient information" is received from the thermometer 10 or not is determined.

If it is determined in ST8 that "patient information" is not received, the process proceeds to ST9.

In ST9, "patient information unregistered" is displayed on the "ward terminal display 84" of the "ward terminal 80" that is being operated by the nurse to urge input of "patient information" from the "ward terminal input device 85". For example, it is urged to input information such as a "patient ID", which is a part of the patient information.

Next, the process proceeds to ST10. In ST10, the "patient information processing unit (program) 88" in Fig. 3 is executed, and whether patient information (whole or partial) is input from the ward terminal input device 85 or not is determined.

If it is determined in ST10 that there is an input of "patient information (whole or partial) ", the process proceeds to ST11.

In ST11, the ward terminal 80 refers to the "ward electronic medical record database 55" of the "ward electronic medical record server 50" in Fig. 4 based on the input "patient information (whole or partial)", and acquires, and transmits to the thermometer 10, "patent information" corresponding to the input "patient information".

Next, the process proceeds to ST12. In ST12, the thermometer 10 stores the received "patient information" in the "patient information storage unit 19" in Fig. 2.

Accordingly, the thermometer 10 may acquire reliable certain patient information from the ward electronic medical record server 50 without possessing the patient information beforehand.

Furthermore, the thermometer 10 does not require authentication or the like of patient information, and thus the manufacturing cost of the thermometer may be reduced.

Furthermore, the thermometer 10 holds only the latest measured body temperature information, and thus there is no confusion about which body temperature information or the like is associated with the patient information acquired from the ward electronic medical record server 50, and there is no mix-up of the patient information and the body temperature information.

The processes of the thermometer 10, the ward terminal 80 and the like for the patient in the ward are then ended.

Then, the patient takes the thermometer 10 to the outpatient clinic to receive consultation in the outpatient clinic.

At this time, a doctor or the like brings the thermometer 10 close to the "outpatient terminal read/write device 91" in Fig. 1.

That is, the step in ST5 in Fig. 5 is started. Then, whether "patient information" is stored in the thermometer 10 or not is determined in ST6.

In this case, "patient information" is already stored in the "patient information storage unit 19" in Fig. 2 in ST12 described above, and thus "YES" is obtained, and the process proceeds to ST13 in Fig. 6.

In ST13, the thermometer 10 transmits, to the "outpatient terminal read/write device 91", the patient information in the patient information storage unit 19 and the "measured body temperature information" that is stored in the "measured body temperature storage unit 16", and these pieces of data are transmitted to the outpatient electronic medical record server 70 via the outpatient terminal 90.

Additionally, the "ward electronic medical record server 50" is an example of "one biological information management device", and the "outpatient electronic medical record server 70" is an example of "another biological information management device".

Accordingly, in the present embodiment, the thermometer 10 measures the body temperature and transmits the measured body temperature information to the ward electronic medical record server 50, and afterwards still holds the same measured body temperature information, and thus the same measured body temperature information may be transmitted afterwards to the outpatient electronic medical record server 70, which is a different server.

Also, at this time, the thermometer 10 holds the patient information together with the measured body temperature information, and this patient information is highly accurate information which is obtained from the ward electronic medical record server 50.

This patient information and the measured body temperature information are transmitted together to the outpatient electronic medical record server 70, and thus pieces of data in the outpatient electronic medical record server 70 and the ward electronic medical record server 50 may be made uniform, and occurrence of a situation where the patient information and the body temperature information stored in the servers are different may be prevented.

The present invention is not limited to the embodiment described above. The present embodiment cites body temperature information as the biological information, but biological information such as blood pressure information, blood sugar level information, body composition information, pulse information and the like may also be handled in the same manner.

### Reference Signs List

1 Biological information management system
10 Thermometer
11 Thermometer-side communication device
12 Thermometer control unit
13 Body temperature measurement device
13a Sensor (thermistor)
14 Thermometer display
15 Thermometer power switch
16 Measured body temperature storage unit
17 Already registered measured body temperature information processing unit (program)
18 Transmission content determination unit (program)
19 Patient information storage unit
50 Ward electronic medical record server
51 Ward electronic medical record server control unit
52 Ward electronic medical record server communication device
53 Ward electronic medical record server display
54 Ward electronic medical record server input device
55 Ward electronic medical record database
70 Outpatient electronic medical record server
80 Ward terminal
81 Ward terminal read/write device
82 Ward terminal control unit
83 Ward terminal communication device
84 Ward terminal display
85 Ward terminal input device
86 Ward various information storage unit
87 Patient information acquisition determination unit (program)
88 Patient information processing unit (program)
90 Outpatient terminal
91 Outpatient terminal read/write device

## Claims

1. A biological information management system comprising:
a biological information acquisition device for storing biological information acquired from a subject person; and
a biological information management device for managing the biological information together with subject person information,
wherein the biological information acquisition device includes a biological information storage unit for storing the biological information, and when new biological information is acquired, the biological information acquisition device causes the biological information storage unit to store the new biological information, and deletes the biological information that is already registered from the biological information storage unit, and
wherein the biological information acquisition device is configured to provide the biological information in response to a request from the biological information management device, and is configured to acquire the subject person information of the provided biological information from the biological information management device.

2. The biological information management system according to claim 1,
wherein the biological information acquisition device is configured to provide the biological information to a plurality of the biological information management devices, and
wherein, when there is a request for the biological information from another biological information management device after the subject person information is acquired from one biological information management device, the biological information acquisition device provides the biological information together with the acquired subject person information.

3. A biological information management method that uses a biological information management system including biological information acquisition device for storing biological information acquired from a subject person and a biological information management device for managing the biological information together with subject person information,
wherein the biological information acquisition device includes a biological information storage unit for storing the biological information, and when new biological information is acquired, the biological information acquisition device causes the biological information storage unit to store the new biological information, and deletes the biological information that is already registered from the biological information storage unit, and
wherein the biological information acquisition device is configured to provide the biological information in response to a request from the biological information management device, and is configured to acquire the subject person information of the provided biological information from the biological information management device.
